(19) Europäisches Patentamt
European Patent Office
Office européen des brevets

(11) Veröffentlichungsnummer: **0 290 906**
**A2**

(12) **EUROPÄISCHE PATENTANMELDUNG**

(21) Anmeldenummer: **88107013.0**

(22) Anmeldetag: **02.05.88**

(51) Int. Cl.[4]: **C07D 405/06 , A01N 43/50 , A01N 43/653 , C07C 131/00 , C07D 307/28**

(30) Priorität: **14.05.87 DE 3716023**

(43) Veröffentlichungstag der Anmeldung:
**17.11.88 Patentblatt 88/46**

(84) Benannte Vertragsstaaten:
**BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Elbe, Hans-Ludwig, Dr.**
**Dasnoeckel 59**
**D-5600 Wuppertal 11(DE)**
Erfinder: **Jautelat, Manfred, Dr.**
**Muellerbaum 28**
**D-5093 Burscheid(DE)**
Erfinder: **Stetter, Jörg, Dr.**
**Gellertweg 4**
**D-5600 Wuppertal 1(DE)**
Erfinder: **Büchel, Karl Heinz, Prof. Dr.**
**Dabringhausener Strasse 42**
**D-5093 Burscheid(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**
Erfinder: **Reinecke, Paul, Dr.**
**Steinstrasse 8**
**D-5090 Leverkusen 3(DE)**

(54) **Azolyl-tetrahydrofuran-2-yliden-methane.**

(57) Neue Azolyl-tetrahydrofuran-2-yliden-methane der Formel

EP 0 290 906 A2

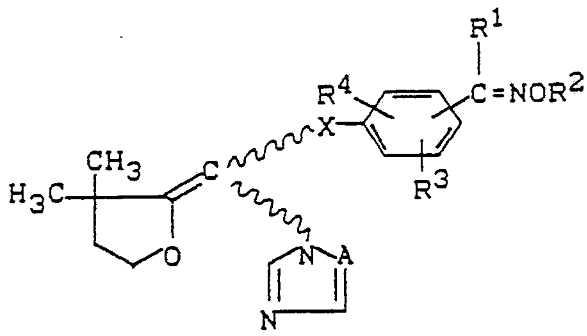

(I)

in welcher
A für ein Stickstoffatom oder die CH-Gruppe steht,
X für Sauerstoff, Schwefel, die SO-oder $SO_2$-Gruppe steht,
$R^1$ für Wasserstoff oder Alkyl steht,
$R^2$ für Wasserstoff oder Alkyl steht und
$R^3$ und $R^4$ unabhängig voneinander für Wasserstoff, Halogen, Alkyl, Alkoxy, Alkylthio, Halogenalkyl, Halogenalkoxy, Halogenalkylthio, Alkoxycarbonyl, Alkoximinoalkyl, Nitro, Cyano, gegebenenfalls durch Halogen und/oder Alkyl substituiertes Phenyl oder für gegebenenfalls durch Halogen und/oder Alkyl substituiertes Phenoxy stehen,
und deren Säureadditions-Salze und Metallsalz-Komplexe,
mehrere Verfahren zur Herstellung der neuen Stoffe und deren Verwendung als Fungizide.
Neue Zwischenprodukte der Formeln

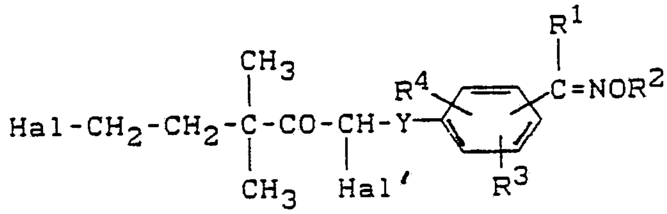

(II)

und

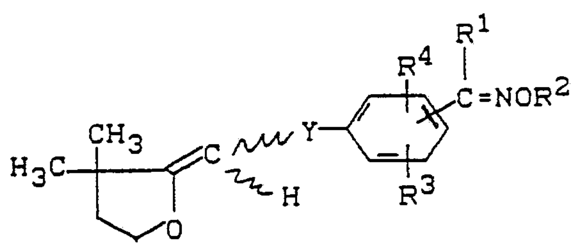

(IX),

Verfahren zu deren Herstellung und deren Verwendung zur Herstellung von Azolyltetrahydrofuran-2-ylidenmethanen der Formel (I).

**Azolyl-tetrahydrofuran-2-yliden-methane**

Die vorliegende Erfindung betrifft neue Azolyl-tetrahydrofuran-2-yliden-methane, mehrere Verfahren zu ihrer Herstellung sowie ihre Verwendung als Fungizide.

Es ist bereits bekannt, daß bestimmte im Phenoxyteil substituierte Azolyl-phenoxy-(3,3-dimethyl-tetrahydrofuran-2-yliden)-methane gute fungizide Eigenschaften aufweisen (vergleiche EP-OS 0 102 517). Die Wirkung dieser Verbindungen ist jedoch, insbesondere bei niedrigen Aufwandmengen und -konzentrationen, nicht immer ganz befriedigend.

Es wurden nun neue Azolyl-tetrahydrofuran-2-yliden-methane der allgemeinen Formel

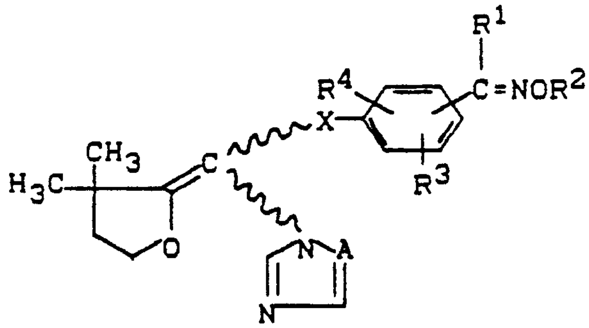

(I)

in welcher
A für ein Stickstoffatom oder die CH-Gruppe steht,
X für Sauerstoff, Schwefel, die SO-oder $SO_2$-Gruppe steht,
$R^1$ für Wasserstoff oder Alkyl steht,
$R^2$ für Wasserstoff oder Alkyl steht und
$R^3$ und $R^4$ unabhängig voneinander für Wasserstoff Halogen, Alkyl, Alkoxy, Alkylthio, Halogenalkyl, Halogenalkoxy, Halogenalkylthio, Alkoxycarbonyl, Alkoximinoalkyl, Nitro, Cyano, gegebenenfalls durch Halogen und/oder Alkyl substituiertes Phenyl oder für gegebenenfalls durch Halogen und/oder Alkyl substituiertes Phenoxy stehen;
und deren Säureadditions-Salze und Metallsalz-Kimplexe gefunden.

Die Verbindungen der Formel (I) können in zwei geometrischen Isomerenformen vorliegen, je nach Anordnung der Gruppen, die an die C = C-Doppelbindung gebunden sind, Vorzugsweise fallen sie in einem Isomerengemisch an. Aufgrund der Oximino-Struktur können die Verbindungen auch in einer syn-oder anti-Form vorliegen, je nach Anordnung der Gruppen die an die C = N-Doppelbindung gebunden sind. Vorzugsweise fallen sie in einem Isomerengemisch an. Die vorliegende Erfindung betrifft sowohl die einzelnen Isomeren als auch die Isomerengemische.

Weiterhin wurde gefunden, daß man die Azolyl-tetrahydrofuran-2-yliden-methane der Formel (I) sowie deren Säureadditions-Salze und Metallsalz-Komplexe erhält, wenn man

a) Halogen-(thio)ether-ketone der Formel

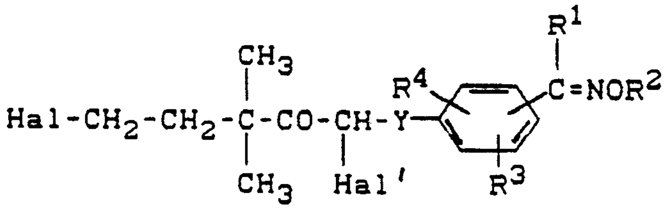

(II)

in welcher
$R^1$, $R^2$, $R^3$ und $R^4$ die oben angegebene Bedeutung haben,
Y für Sauerstoff oder Schwefel steht und
Hal und Hal′ für Halogen stehen,
mit Azolen der Formel

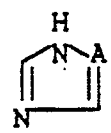

(III)

in welcher A die oben angegebene Bedeutung hat, gegebenenfalls in Gegenwart eines Verdünnmungsmittels und gegebenenfalls in Gegenwart eines Säurebindemittels umsetzt und gegebenenfalls die so erhaltenen Azolyl-tetrahydrofuran-2-yliden-methane der Formel

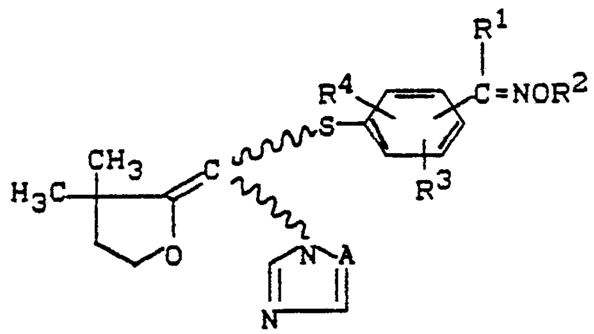

(Ia)

in welcher
A, $R^1$, $R^2$, $R^3$ und $R^4$ die oben angegebene Bedeutung haben,
gegebenenfalls in Gegenwart eines Verdünnungsmittels oxidiert,
oder

b) Halogenketone der Formel

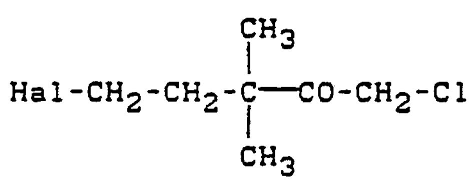

(IV)

in welcher Hal die oben angegebene Bedeutung hat,
mit Azolen der Formel

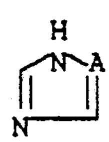

(III)

in welcher
A die oben angegebene Bedeutung hat,
gegebenenfalls in Gegenwart eines Verdünnungsmittels und gegebenenfalls in Gegenwart eines Säurebindemittels umsetzt, dann die dabei anfallenden Azolyltetrahydrofuran-2-yliden-methane der Formel

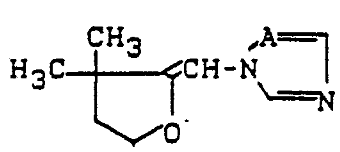

(V)

in welcher
A die oben angegebene Bedeutung hat,
mit Brom in Gegenwart eines Verdünnungsmittels umsetzt und die dabei entstehenden Verbindungen der

Formel

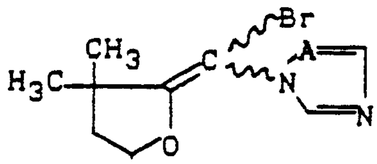

(VI)

in welcher
A die oben angegebene Bedeutung hat,
mit (Thio)Phenolen der Formel

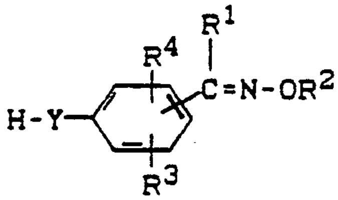

(VII)

in welcher
$R^1$, $R^2$, $R^3$, $R^4$ and Y die oben angegebene Bedeutung haben,
gegebenenfalls in Gegenwart eines Säurebindemittels und gegebenenfalls in Gegenwart eines Verdünnungsmittels umsetzt und gegebenenfalls die so erhaltenen Azolyl-tetrahydrofuran-2-yliden-methane der Formel

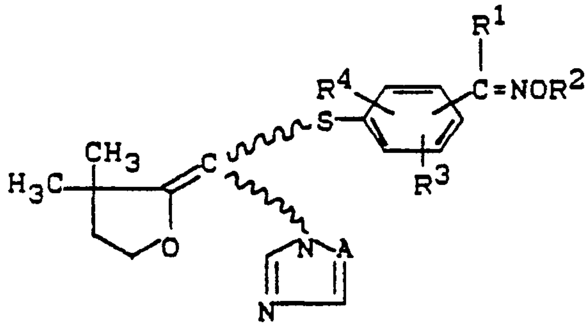

(Ia)

in welcher
A, $R^1$, $R^2$, $R^3$ und $R^4$ die oben angegebene Bedeutung haben,
gegebenenfalls in Gegenwart eines Verdünnungsmittels oxidiert,
oder

c) 2-Chloromethylen-tetrahydrofuran der Formel

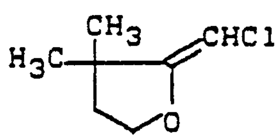

(VIII)

mit (Thio)Phenolen der Formel

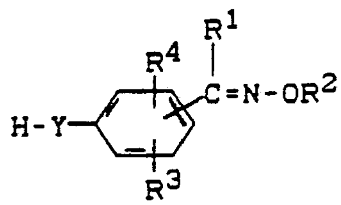

(VII)

in welcher
$R^1$, $R^2$, $R^3$, $R^4$ und Y die oben angegebene Bedeutung haben,
gegebenenfalls in Gegenwart eines Säurebindemittels und gegebenenfalls in Gegenwart eines Verdünnungsmittels umsetzt und danach die so erhaltenen (Thio)-Phenoxytetrahydrofuran-2-yliden-methane der Formel

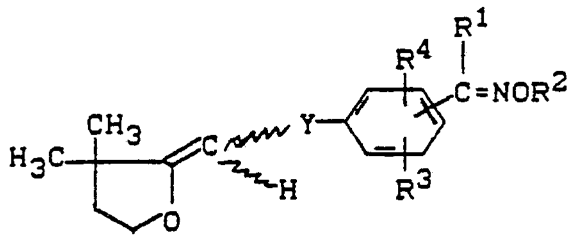

(IX)

in welcher
$R^1$, $R^2$, $R^3$, $R^4$ und Y die oben angegebene Bedeutung haben,
mit Brom in Gegenwart eines Verdünnungsmittels umsetzt und die dabei entstehenden (Thio)Phenoxy-tetrahydrofuran-2-yliden-methane der Formel

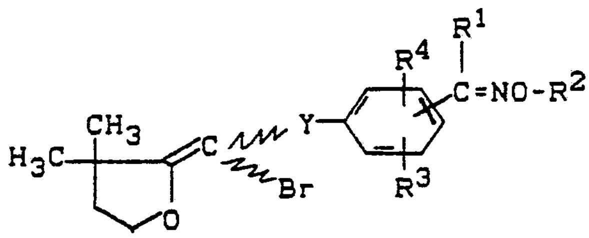

(X)

in welcher
$R^1$, $R^2$, $R^3$, $R^4$ und Y die oben angegebene Bedeutung haben,
mit Azolen der Formel

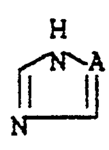

(III)

in welcher
A die oben angegebene Bedeutung hat,
gegebenenfalls in Gegenwart eines Verdünnungsmittels und gegebenenfalls in Gegenwart eines Säurebindemittels umsetzt und gegebenenfalls die so erhaltenen Azolyl-tetrahydrofuran-2-yliden-methane der Formel

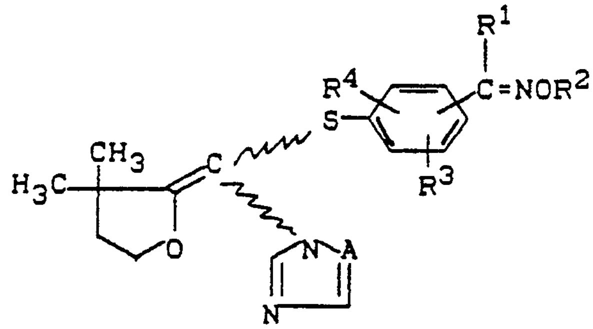

(Ia)

in welcher
A, R', $R^2$, $R^3$ und $R^4$ die oben angegebene Bedeutung haben,
gegebenenfalls in Gegenwart eines Verdünnungsmittels oxidiert, und gegebenenfalls anschließend an die so erhaltenen Verbindungen der Formel (I) eine Säure oder ein Metallsalz addiert.

Schließlich wurde gefunden, daß die neuen Azolyl-tetrahydrofuran-2-yliden-methane der Formel (I) sowie deren Säureadditionssalze und Metallsalz-Komplexe sehr gute fungizide Eigenschaften aufweisen.

Überraschenderweise besitzen die erfindungsgemäßen Stoffe eine bessere fungizide Wirksamkeit als die konstitutionell ähnlichsten vorbekannten Azolyl-phenoxy-(3,3-dimethyltetrahydrofuran-2-yliden)-methane, welches gut wirksame Stoffe gleicher Wirkungsrichtung sind. So übertreffen die erfindungsgemäßen Stoffe beispielsweise das (4-Chlorpenoxy)-(imidazol-1-yl)-(3,3-dimethyltetrahydrofuran-2-yliden)-methan, das (4-Chlorphenoxy)-(1,2,4-triazol-1-yl)-(3,3-dimethyl-tetrahydrofuran-2-yliden)-methan und das (4-Biphenyloxy)-(imidazol-1-yl)-(3,3-dimethyl-tetrahydrofuran-2-yliden)-methan bezüglich der fungiziden Eigenschaften.

Die erfindungsgemäßen Azolyl-tetrahydrofuran-2-ylidenmethane sind durch die Formel (I) allgemein definiert. In dieser Formel stehen vorzugsweise
A für ein Stickstoffatom oder die CH-Gruppe,
X für Sauerstoff, Schwefel, die SO-oder $SO_2$-Gruppe,
$R^1$ für Wasserstoff, Methyl oder Ethyl,
$R^2$ für Wasserstoff oder Alkyl mit 1 bis 4 Kohlenstoffatomen und
$R^3$ und $R^4$ unabhängig voneinander für Wasserstoff, Halogen, Alkyl mit 1 bis 4 Kohlenstoffatomen, Alkoxy und Alkylthio mit jeweils 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro, Cyano, gegebenenfalls durch Halogen und/oder Alkyl mit 1 oder 2 Kohlenstoffatomen substituiertes Phenyl oder für gegebenenfalls durch Halogen und/oder Alkyl mit 1 oder 2 Kohlenstoffatomen substituiertes Phenoxy.

Besonders bevorzugt sind diejenigen Verbindungen der Formel (I), in denen
A für ein Stickstoffatom oder die CH-Gruppe steht,
X für Sauerstoff, Schwefel, die SO-oder $SO_2$-Gruppe steht,
$R^1$ für Wasserstoff, Methyl oder Ethyl steht,
$R^2$ für Wasserstoff, Methyl, Ethyl, n-Propyl, i-Propyl oder n-Butyl steht und
$R^3$ und $R^4$ unabhängig voneinander für Wasserstoff, Fluor, Chlor, Brom. Methyl, Ethyl, tert.-Butyl, Methoxy, Ethoxy, Methylthio, Trifluormethyl, Trifluormethoxy, Trifluormethylthio, Chloridifluormethoxy, Chlordifluormethylthio, Methoxycarbonyl, Ethoxycarbonyl, Methoxyiminomethyl, 1-Methoximinoethyl, Nitro, Cyano, gegebenenfalls durch Chlor und/oder Methyl substituiertes Phenyl oder für gegebenenfalls durch Chlor und/oder Methyl substituiertes Phenoxy stehen.

Ganz besonders bevorzugt sind diejenigen Verbindungen der Formel (I), in denen
A für ein Stickstoffatom oder die CH-Gruppe steht,
X für Sauerstoff, Schwefel, die SO-oder $SO_2$-Gruppe steht,
$R^1$ für Wasserstoff oder Methyl steht,
$R^2$ für Wasserstoff, Methyl, Ethyl, n-Propyl, i-Propyl oder n-Butyl steht und
$R^3$ und $R^4$ unabhängig voneinander für Wasserstoff, Fluor, Chlor, Brom, Methyl, Ethyl, tert-Butyl, Methoxy, Ethoxy, Methylthio, Trifluormelthyl, Trifluormethoxy, Trifluormethylthio, Chlordifluormethoxy, Chlordifluormethylthio, Nitro oder Cyano stehen.

Bevorzugte erfindungsgemäße Verbindungen sind auch Additionsprodukte aus Säuren und denjenigen Azolyl-tetrahydrofuran-2-yliden-methanen der Formel (I), in denen A, X, $R^1$, $R^2$, $R^3$ und $R^4$ diejenigen Bedeutungen haben, die bereits im Zusammenhang mit der Beschreibung der erfindungsgemäßen Stoffe

für diese Substituenten als bevorzugte genannt wurden.

Zu den Säuren, die addiert werden können, gehören vorzugsweise Halogenwasserstoffsäuren, wie z.B. die Chlorwasserstoffsäure und die Bromwasserstoffsäure, ferner Phosphorsäure, Salpetersäure, mono-und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt-und der I. und II. sowie IV. bis VIII. Nebengruppe des Periodensystems der Elemente und denjenigen Azolyl-tetrahydrofuran-2-yliden-methanen der Formel (I), in denen A, X, $R^1$, $R^2$, $R^3$ und $R^4$ diejenigen Bedeutungen haben, die bereits im Zusammenhang mit der Beschreibung der erfindungsgemäßen Stoffe für diese Substituenten als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenphysiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Verbindungen seien außer den bei den Herstellungsbeispielen genannten Verbindungen die in den folgenden Tabellen aufgeführten Stoffe genannt :

<u>Tabelle 1:</u>

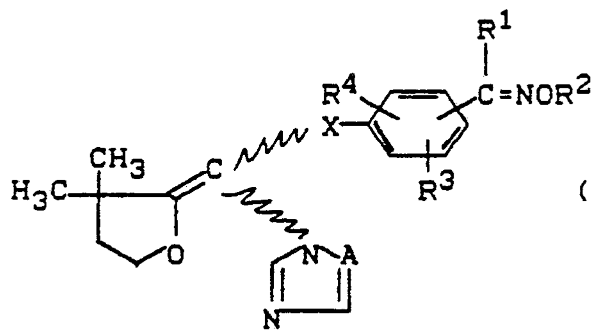

(I)

(X = O, S, SO, $SO_2$ und A = N, CH)

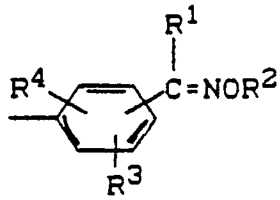

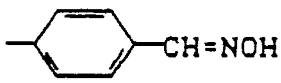
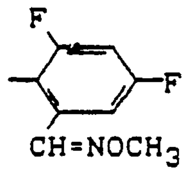
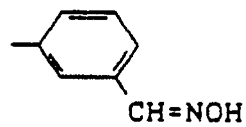

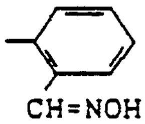
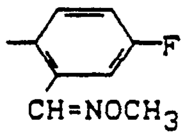
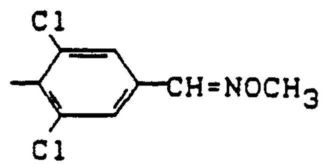

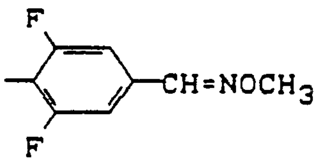
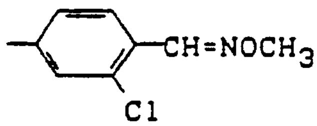
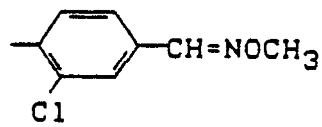

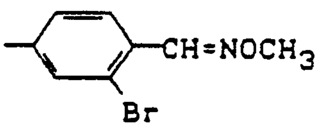
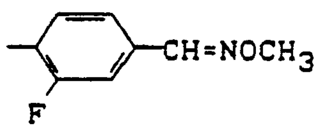
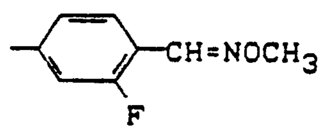

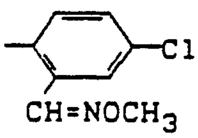
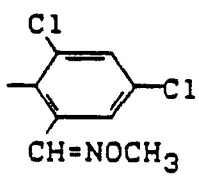
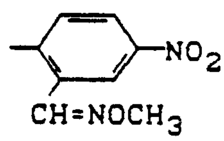

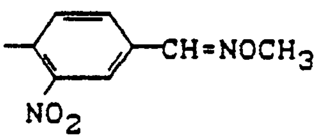
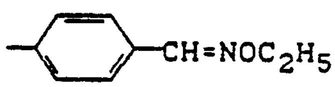
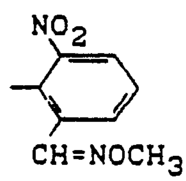

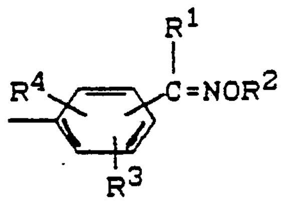

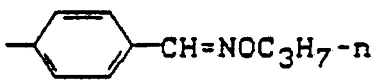
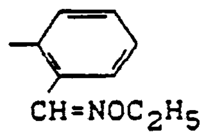
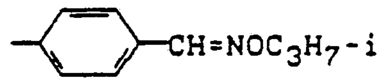

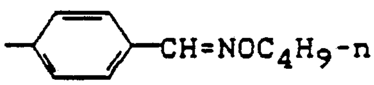
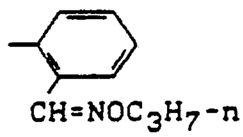
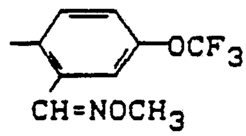

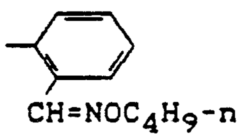
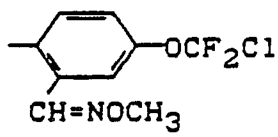
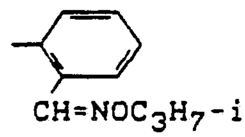

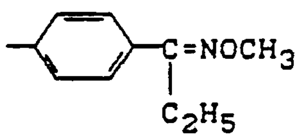
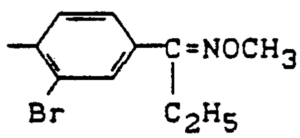
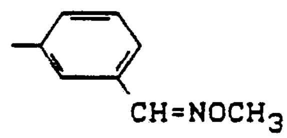

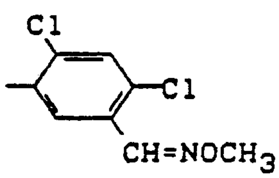
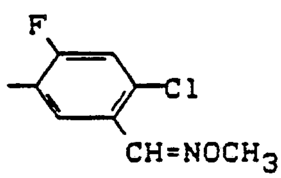
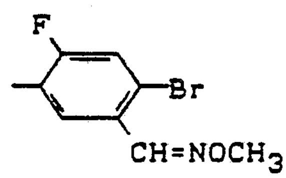

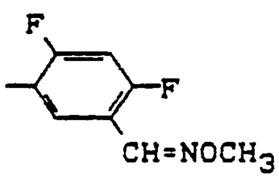
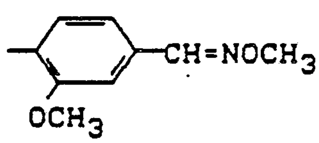
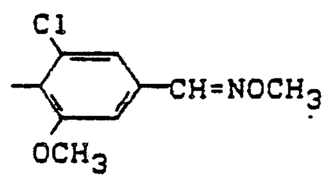

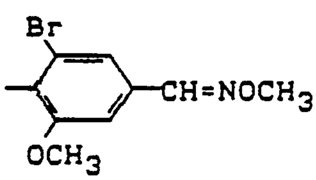
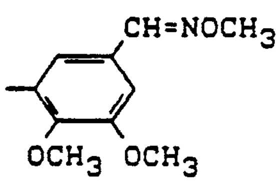
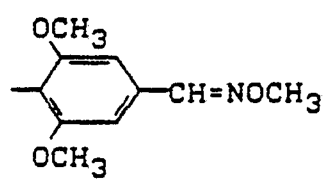

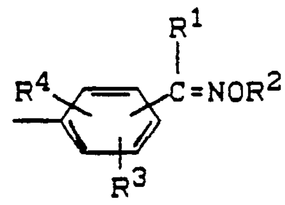

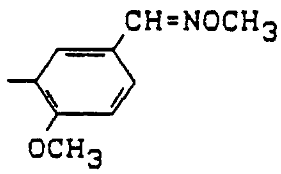

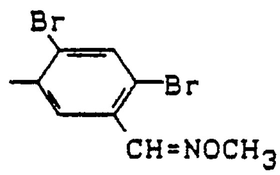

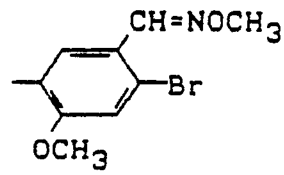

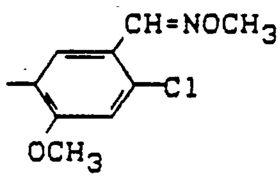

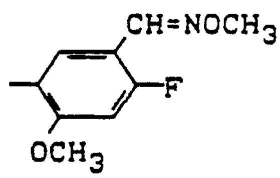

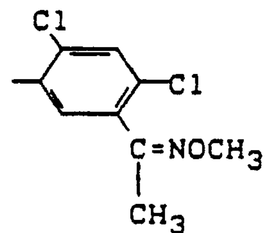

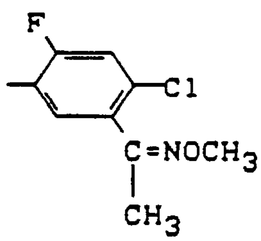

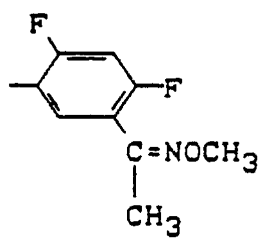

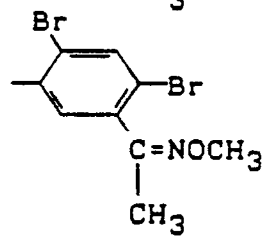

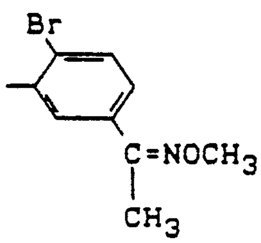

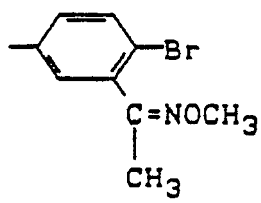

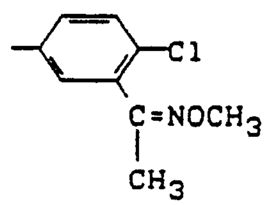

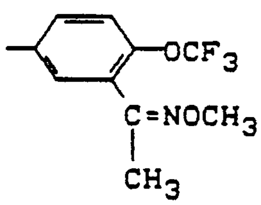

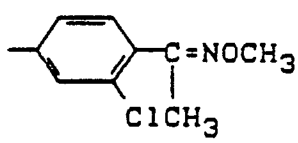

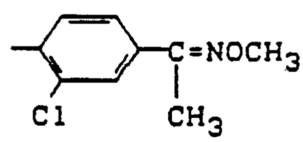

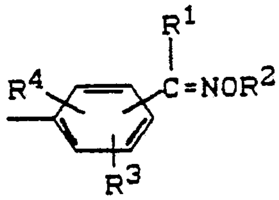

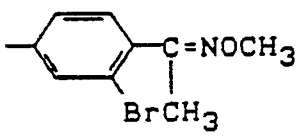

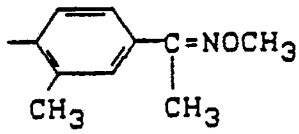

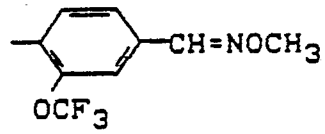

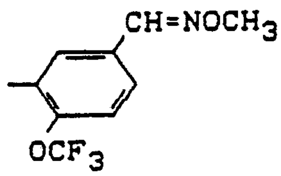

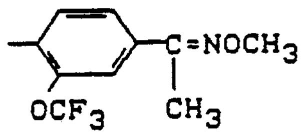

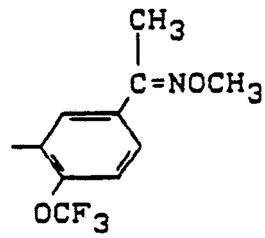

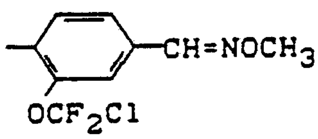

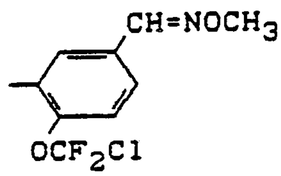

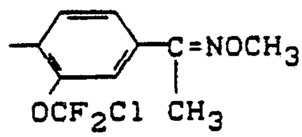

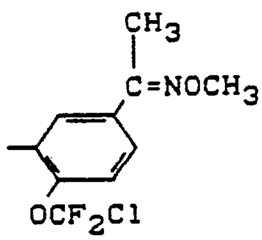

Tabelle 2

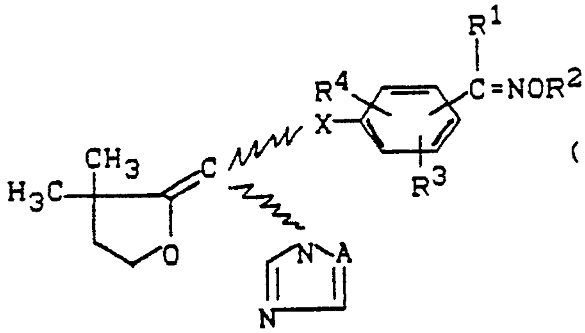

(I)

| 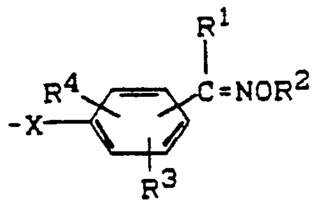 | A |
|---|---|
| 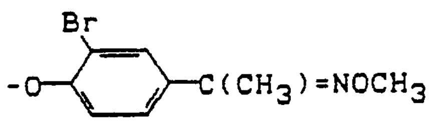 | N |
| 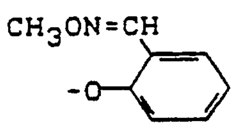 | N |
| 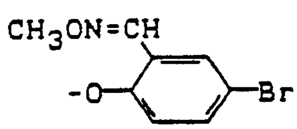 | N |
| 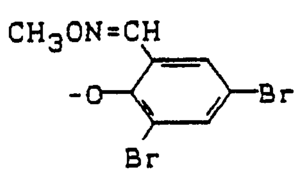 | N |

Tabelle 2 (Fortsetzung)

| Bsp. Nr. | 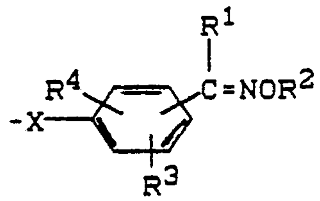 | |
|---|---|---|
| | 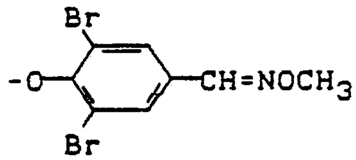 | CH |
| | 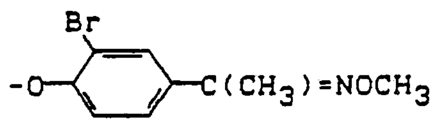 | CH |
| | 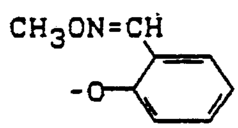 | CH |
| | 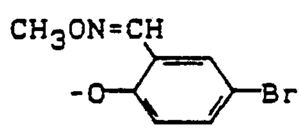 | CH |
| | 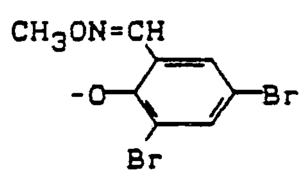 | CH |

Verwendet man beispielsweise 1-Brom-5-chlor-3,3-dimethyl-1-(4-methoximinomethylphenylthio)-2-pentanon und Imidazol als Ausgangsstoffe, so kann der Verlauf des erfindungsgemäßen Verfahrens (a) durch das folgende Formelschema wiedergegeben werden:

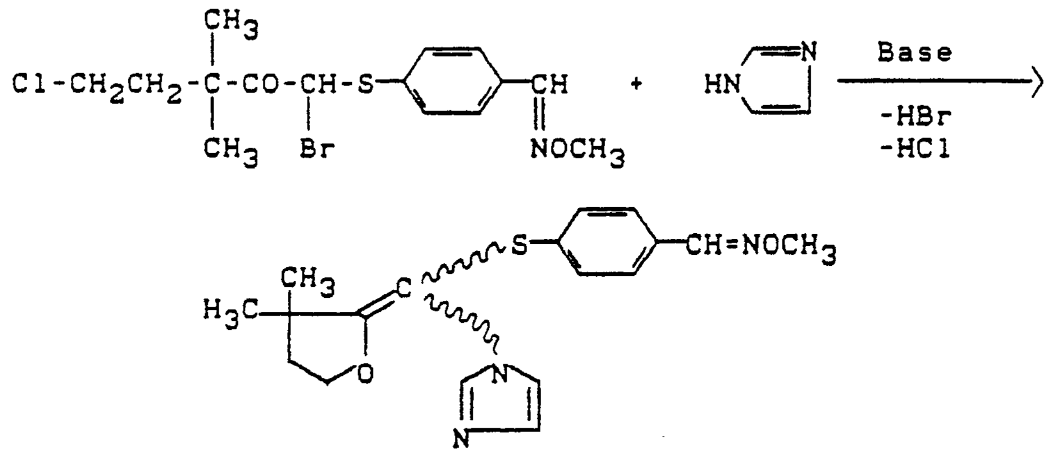


Verwendet man beispielsweise (Imidazol-1-yl)-(4-methoximinomethylphenylthio)-3,3-dimethyltetrahydrofuran-2-yliden-methan als Ausgangsstoff und Wasserstoffperoxid in Eisessig als Oxidationsmittel, so kann der Verlauf der zweiten Stufe des erfindungsgemäßen Verfahrens (a) durch das folgende Formelschema wiedergegeben werden:

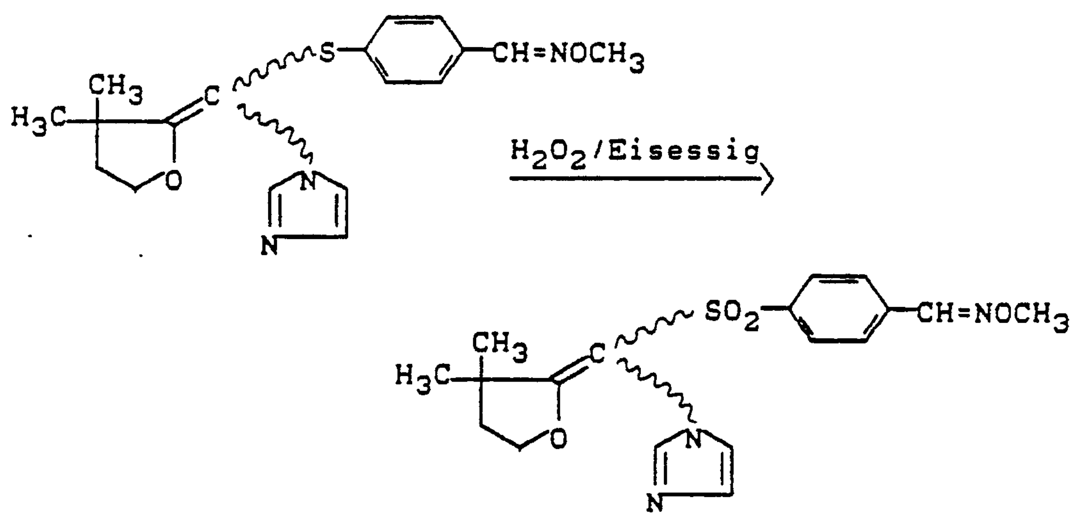


Verwendet man 1,5-Dichlor-3,3-dimethylpentan-2-on und 1,2,4-Triazol als Ausgangsstoffe und Brom sowie 4-Methoximinomethyl-phenol als Reaktionskomponenten, so kann der Verlauf des erfindungsgemäßen Verfahrens (b) durch das folgende Formelschema wiedergegeben werden:

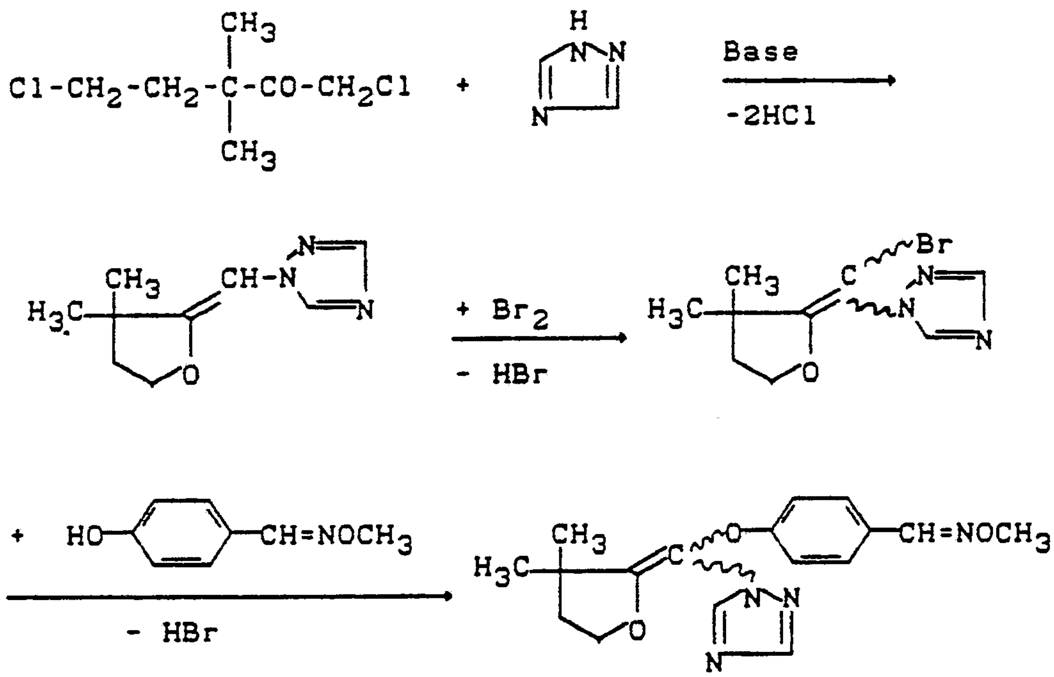


Verwendet man 2-Chlormethylen-tetrahydrofuran und 4-Methoximinomethyl-phenol als Ausgangsstoffe und Brom sowie Imidazol als Reaktionskomponenten, so kann der Verlauf des erfindungsgemäßen Verfahrens (c) durch das folgende Formelschema wiedergegeben werden:

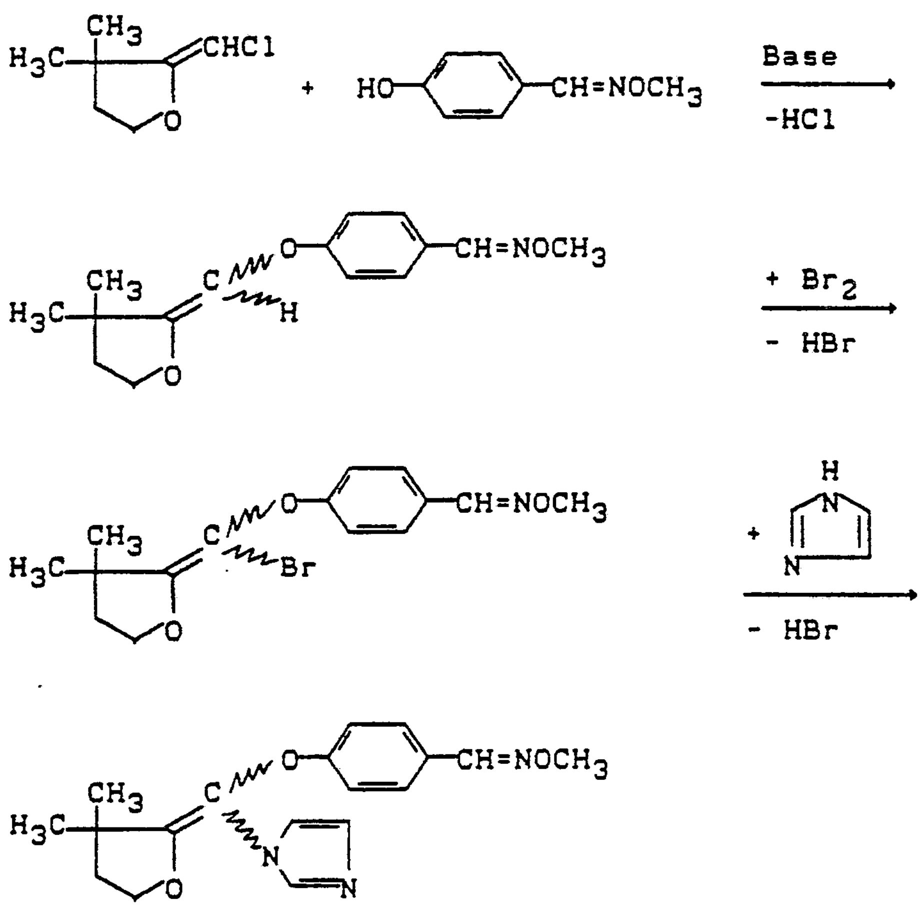

Die bei der Durchführung des erfindungsgemäßen Verfahrens (a) als Ausgangsstoffe zu verwendenden Halogen(thio)etherketone sind durch die Formel (II) allgemein definiert. In dieser Formel stehen $R^1$, $R^2$, $R^3$ und $R^4$ vorzugsweise für die Reste, die bereits im Zusammenhang mit der Beschreibung der erfindungsgemäßen Stoffe der Formel (I) vorzugsweise für diese Substituenten genannt wurden. Hal und Hal' stehen vorzugsweise für Chlor oder Brom.

Die Halogen-(thio)ether-ketone der Formel (II) sind noch nicht bekannt. Sie lassen sich herstellen, indem man Halogenketone der Formel

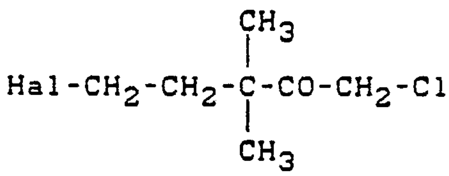 (IV)

in welcher
Hal die oben angegebene Bedeutung hat,
mit (Thio)Phenolen der Formel

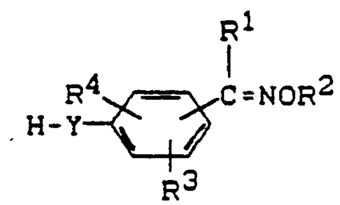 (VII)

in welcher
$R^1$, $R^2$, $R^3$, $R^4$ und Y die oben angegebene Bedeutung haben,
gegebenenfalls in Gegenwart eines Säurebindemittels und gegebenenfalls in Gegenwart eines Verdünnungsmittels umsetzt und die dabei entstehenden (Thio)-Etherketone der Formel

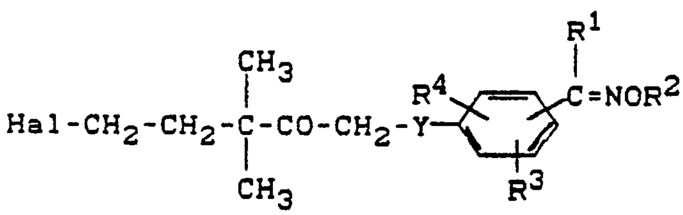 (XI)

in welcher
$R^1$, $R^2$, $R^3$, $R^4$, Hal und Y die oben angegebene Bedeutung haben,
mit Halogen in Gegenwart eines Verdünnungsmittels umsetzt.

Die bei dem obigen Verfahren als Ausgangsstoffe benötigten Halogenketone der Formel (IV) sind bekannt oder lassen sich nach prinzipiell bekannten Verfahren herstellen (vgl. DE-OS 32 04 788). Man erhält die Halogenketone der Formel (IV), indem man Chlormethylen-3,3-dimethyltetrahydrofuran der Formel

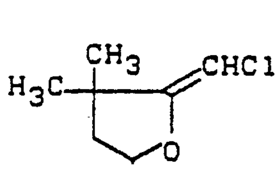 (VIII)

mit Halogenwasserstoffen der Formel
H Hal (XII)
in welcher
Hal die oben angegebene Bedeutung hat,

gegebenenfalls in Gegenwart eines inerten organischen Lösungsmittels, wie beispielsweise Toluol oder Methylenchlorid, bei Temperaturen zwischen 20 und 150 °C umsetzt.

Das 2-Chlormethylen-3,3-dimethyltetrahydrofuran der Formel (VIII) ist ebenfalls bekannt (vergleiche DE-OS 32 04 692). Es wird durch sukzessive Umsetzung von 1,1,5-Trichlor-3,3-dimethyl-1-penten (vergleiche DE-OS 30 29 270) mit Carboxylaten, wie beispielsweise wasserfreiem Natriumacetat, und mit Basen wie beispielsweise Natriummethylat, in Gegenwart eines inerten organischen Lösungsmittels, wie beispielsweise Dimethylformamid, unter Rückflußtemperatur erhalten.

Die bei dem obigen Verfahren weiterhin als Ausgangsstoffe benötigten Phenole bzw. Thiophenole der Formel (VII) sind bekannt oder können nach allgemein bekannten Methoden der organischen Chemie hergestellt werden.

Als Säurebindemittel können bei der Herstellung von Halogen-(thio)ether-ketonen der Formel (II) nach dem obigen Verfahren alle üblichen Säureakzeptoren verwendet werden. Vorzugsweise verwendbar sind Alkalimetallcarbonate, wie Natriumcarbonat und Kaliumcarbonat, ferner Alkalimetallhydrogencarbonate, wie Natriumhydrogencarbonat, weiterhin niedere tertiäre Alkylamine, Cycloalkylamine und Arylalkylamine, wie Triethylamin, N,N-Dimethyl-cyclohexylamin, Dicyclohexylamin, N,N-Dimethyl-benzylamin, und auch aromatische und bicyclische Amine, wie Pyridin und Diaza-bicyclo-octan.

Als Verdünnungsmittel kommen bei dem obigen Verfahren zur Herstellung von Halogen-(thio)ether-ketonen der Formel (II) alle üblichen inerten organischen Solventien in Frage. Vorzugsweise verwendbar sind Ketone, wie Diethylketon, Methyl-ethyl-keton und Aceton, ferner Nitrile, wie Propionitril und Acetonitril, außerdem Alkohole, wie Methanol, Ethanol und Isopropanol, weiterhin Ether, wie Diethylether, Tetrahydrofuran und Dioxan, darüber hinaus aromatische Kohlenwasserstoffe, wie Benzol, Toluol, Xylol und Chlorbenzol, ferner Amide, wie Dimethylformamid, sowie auch halogenierte Kohlenwasserstoffe, wie Chloroform, Tetrachlorkohlenstoff und Methylenchlorid.

Bei der Herstellung von Halogen-(thio)ether-ketonen der Formel (II) nach dem obigen Verfahren kommen bei der Durchführung der zweiten Stufe als Halogene vorzugsweise Chlor und Brom in Betracht.

Als Verdünnungsmittel kommen bei der Durchführung der zweiten Stufe des obigen Verfahrens zur Herstellung von Halogen-(thio)ether-ketonen der Formel (II) alle für derartige Halogenierungen an Kohlenstoffatomen mit aziden Wasserstoffatomen üblichen organischen Solventien in Frage. Vorzugsweise verwendbar sind halogenierte Kohlenwasserstoffe, wie Chloroform, Tetrachlorkohlenstoffe und Methylenchlorid.

Die Reaktionstemperaturen können bei der Durchführung des obigen Verfahrens zur Herstellung von Halogen-(thio)ether-ketonen der Formel (II) in einem größeren Bereich variiert werden. Im allgemeinen arbeitet man bei der Durchführung der ersten Stufe bei Temperaturen zwischen 0 °C und 150 °C, vorzugsweise zwischen 20 °C und 120 °C. In der zweiten Stufe arbeitet man im allgemeinen bei Temperaturen zwischen 0 °C und 50 °C, vorzugsweise bei Raumtemperatur.

Bei der Durchführung des Verfahrens zur Herstellung von Halogen-(thio)ether-ketonen der Formel (II) setzt man die Reaktionskomponenten im allgemeinen in äquivalenten Mengen ein. Es ist jedoch auch möglich die eine oder andere Komponente in einem Überschuß zu verwenden. Die Aufarbeitung und die Isolierung der gewünschten Stoffe erfolgt jeweils nach üblichen Methoden. Die Halogen-(thio)ether-ketone der Formel (II) können gegebenenfalls ohne Isolierung direkt weiter umgesetzt werden.

Die bei dem erfindungsgemäßen Verfahren (a) in der ersten Stufe als Reaktionskomponenten benötigten Azole der Formel (III) sind bekannt.

Als Säurebindemittel können bei der Durchführung der ersten Stufe des erfindungsgemäßen Verfahrens (a) alle üblichen Säureakzeptoren eingesetzt werden. Vorzugsweise verwendbar sind Alkalimetallcarbonate, wie Natriumcarbonat und Kaliumcarbonat, ferner Alkalimetallhydrogencarbonate, wie Natriumhydrogencarbonat, weiterhin niedere tertiäre Alkylamine, Cycloalkylamine und Arylalkylamine, wie Triethylamin, N,N-Dimelthyl-cyclohexylamin, Dicyclohexylamin, N,N-Dimethyl-benzylamin und auch aromatische und bicyclische Amine, wie Pyridin und Diaza-bicyclo-octan. Es ist jedoch auch möglich, Azol der Formel (III) gleichzeitig als Säurebindemittel zu benutzen.

Als Verdünnungsmittel kommen bei der Durchführung der ersten Stufe des erfindungsgemäßen Verfahrens (a) alle üblichen inerten organischen Solventien in Frage, Vorzugsweise verwendbar sind Ketone, wie Diethylketon, Methyl-ethylketon und Aceton, ferner Nitrile, wie Propionitril und Acetonitril, außerdem Alkohole, wie Methanol, Ethanol und Isopropanol, weiterhin Ether, wie Diethylether, Tetrahydrofuran und Dioxan, darüber hinaus aromatische Kohlenwasserstoffe, wie Benzol, Toluol, Xylol und Chlorbenzol, ferner Amide, wie Dimethylformamid, sowie auch halogenierte Kohlenwasserstoffe, wie Chloroform, Tetrachlorkohlenstoff und Melthylenchlorid.

Die Reaktionstemperaturen können bei der Durchführung der ersten Stufe des erfindungsgemäßen Verfahrens (a) innerhalb eines größeren Bereiches variiert werden. Im allgemeinen arbeitet man bei Temperaturen zwischen 20 °C und 150 °C, vorzugsweise zwischen 60 und 120 °C.

Bei der Durchführung der ersten Stufe des erfindungsgemäßen Verfahrens (a) setzt man auf 1 Mol an Halogen-(thio)ether-keton der Formel (II) vorzugsweise 1 bis 4 Mol Azol der Formel (III) sowie gegebenenfalls 1 bis 4 Mol Säurebindemittel ein. Die Aufarbeitung erfolgt nach üblichen Methoden. Im allgemeinen geht man so vor, daß man das Verdünnungsmittel abdestilliert, den verbleibenden Rückstand in einem mit Wasser wenig mischbaren organischen Lösungsmittel aufnimmt, die entstehende Lösung mit Wasser wäscht und nach dem Trocknen einengt.

Als Oxidationsmittel kommen bei der Durchführung der zweiten Stufe des erfindungsgemäßen Verfahrens (a) alle für derartige Umsetzungen üblichen Oxidatien in Betracht. Vorzugsweise verwendbar sind m-Chlorbenzoesäure oder Wasserstoffperoxid.

Als Verdünnungsmittel kommen bei der Durchführung der zweiten Stufe des erfindungsgemäßen Verfahrens (a) alle für derartige Umsetzungen üblichen organischen Solventien in Frage. Verwendet man m-Chlorperbenzoesäure als Oxidationsmittel, so arbeitet man vorzugsweise in Gegenwart von Methylenchlorid. Setzt man Wasserstoffperoxid als Oxidationsmittel ein, so arbeitet man vorzugsweise in Gegenwart von Essigsäure oder Acetanhydrid.

Die Reaktionstemperaturen können bei der Durchführung der zweiten Stufe des erfindungsgemäßen Verfahrens (a) ebenfalls innerhalb eines größeren Bereiches variiert werden. Im allgemeinen arbeitet man bei Temperaturen zwischen -50 °C und +100 °C, vorzugsweise zwischen -30 °C und +80 °C.

Bei der Durchführung der zweiten Stufe des erfindungsgemäßen Verfahrens (a) setzt man auf 1 Mol an Azolyltetrahydrofuran-2-yliden-methan der Formel (Ia) im allgemeinen 1 bis 5 Mol an Oxidationsmittel ein. Bei der Anwendung von 1 Mol Oxidationsmittel und Reaktionstemperaturen zwischen -30 °C und +30 °C entstehen vorzugsweise diejenigen Verbindungen der Formel (I), in denen X für SO steht. Verwendet man einen überschuß an Oxidationsmittel und arbeitet man bei Temperaturen zwischen 10 °C und 80 °C, so entstehen vorzugsweise diejenigen Verbindungen der Formel (I), in denen X für $SO_2$ steht. Die Aufarbeitung erfolgt nach üblichen Methoden.

Bei der Durchführung der ersten Stufe des erfindungsgemäßen Verfahrens (b) entsprechen die Reaktionsbedingungen denjenigen der ersten Stufe des erfindungsgemäßen verfahrens (a). Die dabei entstehenden Azolyl-tetrahydrofuran-2-ylidenmethane der Formel (V) sind bekannt (vgl. DE-OS 32 04 795).

Bei der Durchführung der zweiten Stufe des erfindungsgemäßen Verfahrens (b) kommen als Verdünnungsmittel alle für derartige Umsetzungen üblichen inerten organischen Solventien in Betracht. Vorzugsweise verwendbar sind halogenierte Kohlenwasserstoffe, wie Chloroform, Tetrachlorkohlenstoff und Methylenchlorid.

Die Reaktionstemperaturen können bei der Durchführung der zweiten Stufe des erfindungsgemäßen Verfahrens (b) innerhalb eines bestimmten Bereiches variiert werden. Im allgemeinen arbeitet man bei Temperaturen zwischen 0 und 80 °C, vorzugsweise bei Raumtemperatur.

Bei der Durchführung der zweiten Stufe des erfindungsgemäßen Verfahrens arbeitet man nach üblichen Methoden. Die Aufarbeitung erfolgt ebenfalls in bekannter Weise.

Bei der Durchführung der dritten Stufe des erfindungsgemäßen Verfahrens (b) entsprechen die Reaktionsbedingungen denjenigen der ersten Stufe des erfindungsgemäßen Verfahrens (a).

In der vierten Stufe des erfindungsgemäßen Verfahrens (b) erfolgt die Oxidation in gleicher Weise wie in der zweiten Stufe des erfindungsgemäßen Verfahrens (a).

Bei der Durchführung der ersten Stufe des erfindungsgemäßen Verfahrens (c) entsprechen die Reaktionsbedingungen denjenigen der ersten Stufe des erfindungsgemäßen Verfahrens (a). Die dabei entstehenden (Thio)-Phenoxytetrahydrofuran-2-yliden-methane der Formel (IX) sind bisher noch nicht bekannt.

Bei der Durchführung der zweiten Stufe des erfindungsgemäßen Verfahrens (c) arbeitet man in gleicher Weise wie bei der zweiten Stufe des erfindungsgemäßen Verfahrens (b).

Bei der Durchführung der dritten Stufe des erfindungsgemäßen Verfahrens (c) entsprechen die Reaktionsbedingungen denjenigen der ersten Stufe des erfindungsgemäßen Verfahrens (a).

In der vierten Stufe des erfindungsgemäßen Verfahrens (c) erfolgt die Oxidation in gleicher Weise wie in der zweiten Stufe des erfindungsgemäßen Verfahrens (a).

Die nach den erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditionssalze oder Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in

bekannter Weise, z.B. durch Abfiltrieren isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metall salz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen als Fungizide und Bakterizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die erfindungsgemäßen Wirkstoffe können mit besonders gutem Erfolgt zur Bekämpfung von Botrytis; von Getreidekrankheiten, wie insbesondere Puccinia recondita, Pyrenophora teres, Fusarium culmorum und Leptosphaeria nodorum, sowie von Reiskrankheiten, wie Pyricularia oryzae eingesetzt werden.
Dabei zeigen die erfindungsgemäßen Stoffe neben guter in-vivo-Wirkung auch eine gute in-vitro-Wirkung.

In entsprechenden Aufwandmengen zeigen die erfindungsgemäßen Wirkstoffe auch pflanzenwachstumsregulierende Eigenschaften.

Die Wirkstoffe können in die üblichen Formulierungen übergeführt werden, wie Lösungen, Emulsionen, Suspensionen, Pulver, Schäume, Pasten, Granulate, Aerosole. Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmittel, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als

flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier-und/oder - schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Wirkstoffe geht aus den folgenden Beispielen hervor.

Herstellungsbeispiele

Beispiel 1

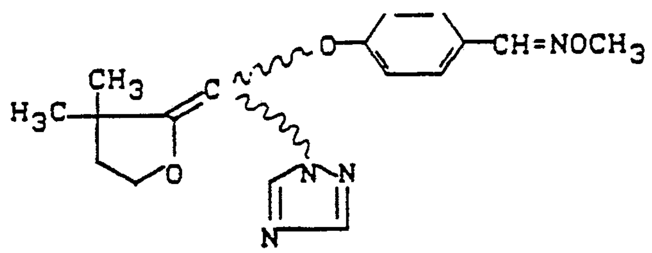

(I-1)

116 g (0,308 Mol) 1-Brom-5-chlor-3,3-dimethyl-1-(4-methoximinomethylphenoxy)-2-pentanon, 25,5 g (0,37 Mol) 1,2,4-Triazol und 127,5 g (0,924 Mol) Kaliumcarbonat werden in 1,5 Liter Aceton 10 Stunden unter Rückfluß erhitzt. Man läßt auf Raumtemperatur abkühlen und saugt vom festen Rückstand ab. Das Filtrat wird eingeengt, in Methylenchlorid aufgenommen, mit Wasser gewaschen, über Natriumsulfat getrocknet und erneut eingeengt. Der Rückstand wird säulenchromatographisch gereinigt (Kieselgel; Essigester/Cyclohexan = 3 : 1). Man erhält 20,7 g (20,4 % der Theorie) (4-Methoximinomethylphenoxy)-(1,2,4-triazol-1-yl)-(3,3-dimethyltetrahydrofuran-2-yliden)-methan vom Brechungsindex $n_D^{20}$ = 1,5612.

Herstellung der Ausgangsprodukte

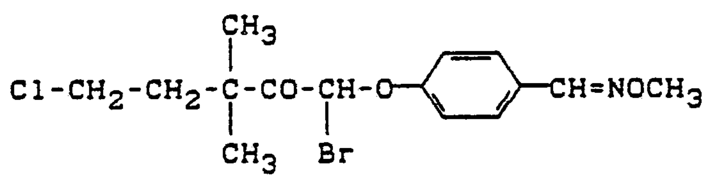

(II-1)

179 g (0,6 Mol) 5-Chlor-3,3-dimethyl-1-(4-methoximinomethylphenoxy)-2-pentanon werden in 600 ml Chloroform vorgelegt. Man läßt bei Raumtemperatur 96,2 g (0,6 Mol) Brom so zutropfen, daß sich die Lösung immer entfärbt. Anschließend wird 1 Stunde bei Raumtemperatur nachgerührt und das Reaktionsgemisch wird durch Abdestillieren des Lösungsmittels eingeengt. Man erhält 233,2 g (100 % der Theorie) 1-Brom-5-chlor-3,3dimethyl-1-(4-methoximinomethylphenoxy)-2-pentanon vom Brechungsindex $n_D^{20}$ = 1,5497.

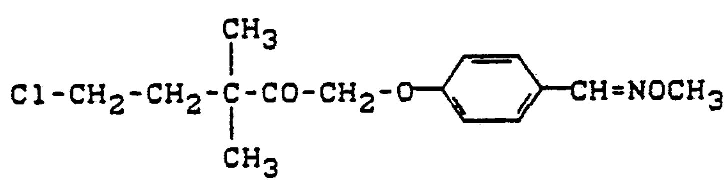

(X-1)

129 g (0,854 Mol) 4-Methoximinomethylphenol und 117,9 g (0,854 Mol) Kaliumcarbonat in 850 ml Toluol werden zwei Stunden unter Rückfluß am Wasserabscheider erhitzt. Bei 100° C versetzt man anschließend mit 130,2 g (0,712 Mol) 1,5-Dichlor-3,3-dimethyl-2-pentanon in 200 ml Toluol. Das Reaktionsgemisch wird 6 Stunden bei 100° C nachgerührt. Danach läßt man auf Raumtemperatur abkühlen und verrührt mit 800 ml Wasser. Die organische Phase wird abgetrennt, mit verdünnter, wäßriger Natronlauge und mit Wasser gewaschen, über Natriumsulfat getrocknet und eingeengt. Man erhßlt 179,6 g (84,7 % der Theorie) 5-Chlor-3,3-dimethyl-1-(4-methoximinomethylphenoxy)-2-pentanon vom Brechungsindex $n_D^{20}$ = 1,5394.

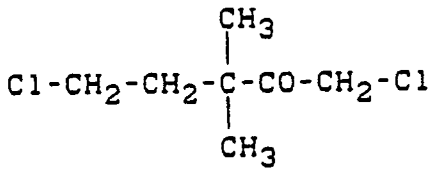

(IV-1)

In 476 g (3,25 Mol) 2-Chlormethylen-3,3-dimethyltetrahydrofuran wird unter Eiskühlung ein starker Chlorwasserstoffgasstrom aus einer Bombe eingeleitet. Das Gas wird vollständig absorbiert und die Innentemperatur steigt bis auf 30° C. Nach vollständiger Sättigung mit Chlorwasserstoff wird das Reaktionsgemisch 2 Stunden bei Raumtemperatur nachgerührt. Überschüssiger Chlorwasserstoff wird zunächst im Wasserstrahlpumpen-Vakuum abgezogen, dann wird im Hochvakuum destilliert. Man erhält 531 g (90 % der Theorie) 1,5-Dichlor-3,3-dimethyl-2-pentanon vom Siedepunkt 85-90° C/0,3 mbar.

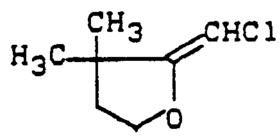

(VIII-1)

806 g (4 Mol) 1,1,5-Trichlor-3,3-dimethyl-1-penten werden mit 360 g (4,4 Mol) wasserfreiem Natriumacetat in 1000 ml Dimethylformamid 6 Stunden unter Rückfluß erhitzt. Nach Abkühlung auf 100° C tropft man 1,6 l (8 Mol) 30%-ige Natriummethylatlösung in Methanol ein und erhitzt weitere 4 Stunden unter Rückfluß. Die kalte Lösung wird in Wasser gegossen und mit Methylenchlorid mehrfach extrahiert.

Nach Trocknen der organischen Phase und Abdesillieren des Lösungsmittels bleiben 654 g Produkt zurück, das über eine Kolonne fraktioniert wird. Man erhält 522 g (89 % der Theorie) 2-Chlormethylen-3,3-dimethyltetrahydrofuran vom Siedepunkt 84-87° C/20 mbar.

In entsprechender Weise und gemäß den angegebenen Verfahrensbedingungen werden die folgenden Verbindungen der Formel

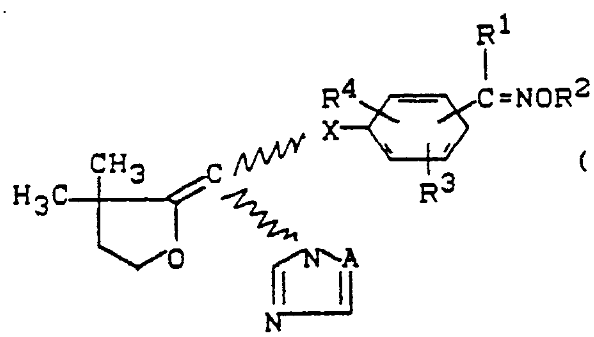

(I)

hergestellt:

| Bsp. Nr. | 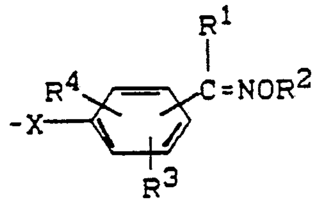 | A | Physikal. Konst. |
|---|---|---|---|
| 2 | 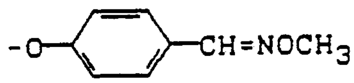 | CH | Fp: 117-119° C |
| 3 | 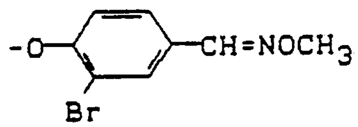 | CH | $n_D^{20}$ : 1,5861 |
| 4 | 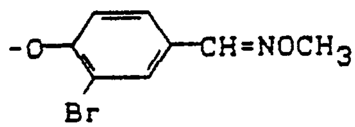 | N | $n_D^{20}$ : 1,5889 |
| 5 | 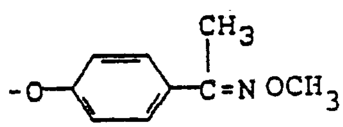 | CH | Fp: 103-106° C |
| 6 | 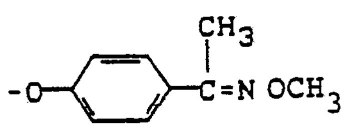 | N | $n_D^{20}$ : 1,5530 |
| 7 | 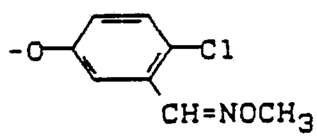 | CH | $n_D^{20}$ : 1,5562 |

Anwendungsbeispiele:

In den folgenden Anwendungsbeispielen werden die nachstehend aufgeführten Verbindungen als Vergleichssubstanzen verwendet:

(A)

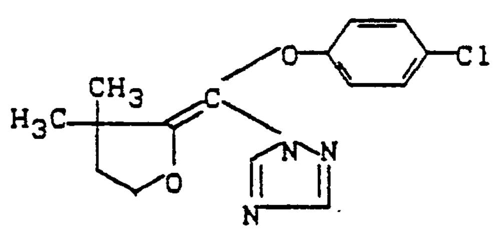

(4-Chlorphenoxy)-(1,2,4-triazol-1-yl)-(3,3-dimethyltetrahydrofuran-2-yliden)-methan

(B)

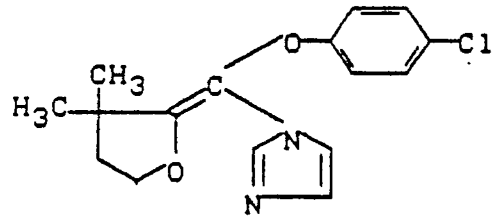

(4-Chlorphenoxy)-(imidazol-1-yl)-(3,3-dimethyltetrahydrofuran-2-yliden)-methan
(C)

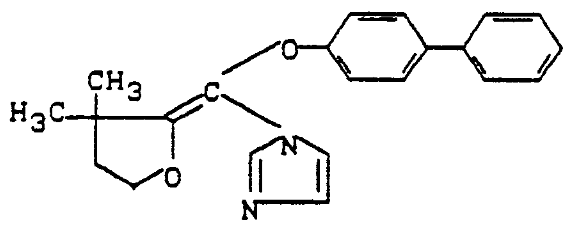

(4-Biphenyloxy)-(imidazol-1-yl)-(3,3-dimethyltetrahydrofuran-2-yliden)-methan (vgl EP-OS 0 102 517)

Beispiel A

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

In diesem Test zeigen die erfindungsgemäßen Stoffe gemäß Beispielen 1 und 2 eine wesentlich bessere Wirksamkeit als die Vergleichssubstanzen (A), (B) und (C).

Beispiel B

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

In diesem Test zeigen die Wirkstoffe gemäß Beispielen 1 und 2 eine wesentlich bessere Wirksamkeit als die Vergleichssubstanz (A).

## Ansprüche

1. Azolyl-tetrahydrofuran-2-yliden-methane der Formel

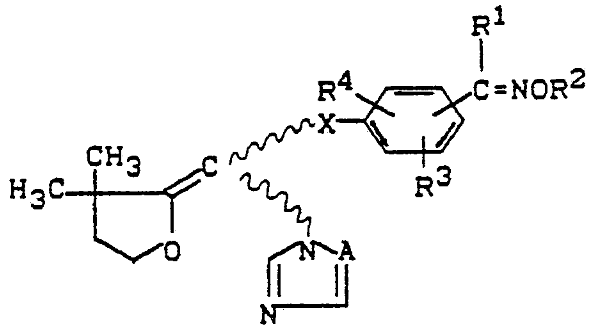

(I)

in welcher
A für ein Stickstoffatom oder die CH-Gruppe steht,
X für Sauerstoff, Schwefel, die SO-oder $SO_2$-Gruppe steht,
$R^1$ für Wasserstoff oder Alkyl steht,
$R^2$ für Wasserstoff oder Alkyl steht und
$R^3$ und $R^4$ unbhängig voneinander für Wasserstoff, Halogen, Alkyl, Alkoxy, Alkylthio, Halogenalkyl, Halogenalkoxy, Halogenalkylthio, Alkoxycarbonyl, Alkoximinoalkyl, Nitro, Cyano, gegebenenfalls durch Halogen und/oder Alkyl substituiertes Phenyl oder für gegebenenfalls durch Halogen und/oder Alkyl substituiertes Phenoxy stehen,
und deren Säureadditions-Salze und Metallsalz-Komplexe.

2. Azolyl-tetrahydrofuran-2-yliden-methane der Formel (I) gemäß Anspruch 1, in denen
A für ein Stickstoffatom oder die CH-Gruppe steht,
X für Sauerstoff, Schwefel, die SO-oder $SO_2$-Gruppe steht,
$R^1$ für Wasserstoff, Methyl oder Ethyl steht,
$R^2$ für Wasserstoff oder Alkyl mit 1 bis 4 Kohlenstoffatomen und
$R^3$ und $R^4$ unabhängig voneinander für Wassertoff, Halogen, Alkyl mit 1 bis 4 Kohlenstoffatomen, Alkoxy und Alkylthio mit jeweils 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschidenen Halogenatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro, Cyano, gegebenenfalls durch Halogen und/oder Alkyl mit 1 oder 2 Kohlenstoffatomen substituiertes Phenyl oder für gegebenenfalls durch Halogen und/oder Alkyl mit 1 oder 2 Kohlenstoffatomen substituiertes Phenoxy stehen.

3. Verfahren zur Herstellung von Azolyl-tetrahydrofuran-2-yliden-methanen der Formel

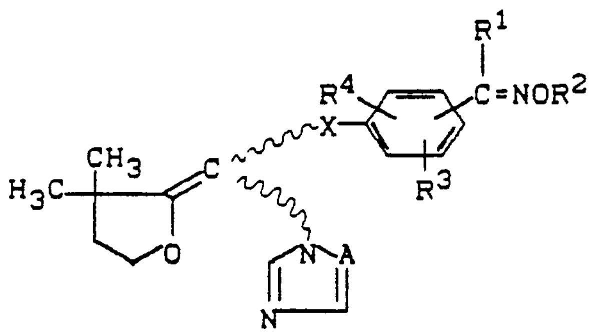

(I)

in welcher
A für ein Stickstoffatom oder die CH-Gruppe steht,
X für Sauerstoff, Schwefel, die SO-oder $SO_2$-Gruppe steht,
$R^1$ für Wasserstoff oder Alkyl steht,
$R^2$ für Wasserstoff oder Alkyl steht und
$R^3$ und $R^4$ unabhängig voneinander für Wasserstoff, Halogen, Alkyl, Alkoxy, Alkylthio, Halogenalkyl, Halogenalkoxy, Halogenalkylthio, Alkoxycarbonyl, Alkoximinoalkyl, Nitro, Cyano, gegebenenfalls durch Halogen und/oder Alkyl substituiertes Phenyl oder für gegebenenfalls durch Halogen und/oder Alkyl substituiertes Phenoxy stehen,
sowie von deren Säureadditions-Salzen und Metallsalz-Komplexen, dadurch gekennzeichnet, daß man
a) Halogen-(thio)ether-ketone der Formel

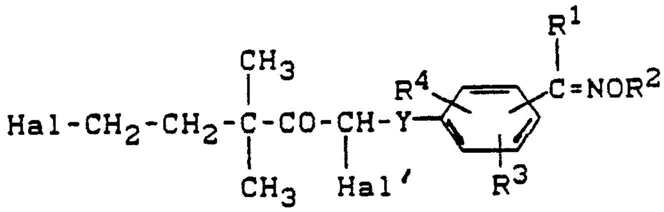

(II)

in welcher
$R^1$, $R^2$, $R^3$ und $R^4$ die oben angegebene Bedeutung haben,
Y für Sauerstoff oder Schwefel steht und
Hal und Hal' für Halogen stehen,
mit Azolen der Formel

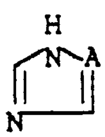

(III)

in welcher
A die oben angegebene Bedeutung hat,
gegebenenfalls in Gegenwart eines Verdünnungsmittels und gegebenenfalls in Gegenwart eines Säurebindemittels umsetzt und gegebenenfalls die so erhaltenen Azolyl-tetrahydrofuran-2-yliden-methane der Formel

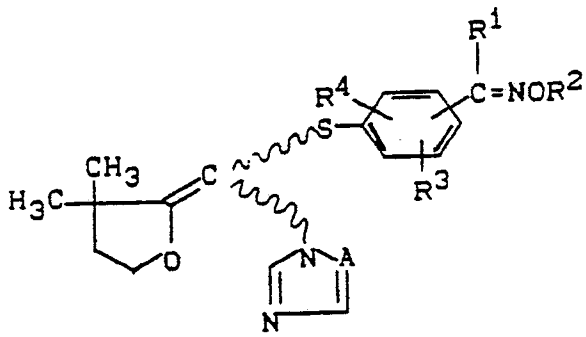

(Ia)

in welcher
A, $R^1$, $R^2$, $R^3$ und $R^4$ die oben angegebene Bedeutung haben,
gegebenenfalls in Gegenwart eines Verdünnungsmittels oxidiert,
oder
b) Halogenketone der Formel

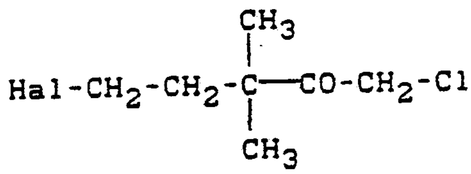

(IV)

in welcher
Hal die oben angegebene Bedeutung hat,
mit Azolen der Formel

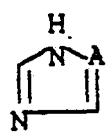

(III)

in welcher
A die oben angegebene Bedeutung hat,
gegebenenfalls in Gegenwart eines Verdünnungsmittels und gegebenenfalls in Gegenwart eines Säurebindemittels umsetzt, dann die dabei anfallenden Azolyl-tetrahydrofuran-2-yliden-methane der Formel

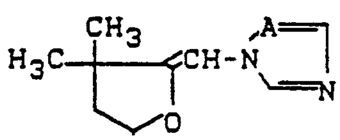

(V)

in welcher
A die oben angegebene Bedeutung hat,
mit Brom in Gegenwart eines Verdünnungsmittels umsetzt und die dabei entstehenden Verbindungen der Formel

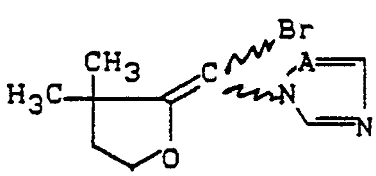

(VI)

in welcher

A die oben angegebene Bedeutung hat, mit (Thio)Phenolen der Formel

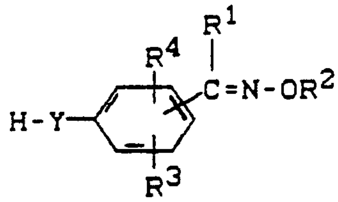

(VII)

in welcher
$R^1$, $R^2$, $R^3$, $R^4$ und Y die oben angegebene Bedeutung haben,
gegebenenfalls in Gegenwart eines Säurebindemittels und gegebenenfalls in Gegenwart eines Verdünnungsmittels umsetzt und gegebenenfalls die so erhaltenen Azolyl-tetrahydrofuran-2-yliden-methane der Formel

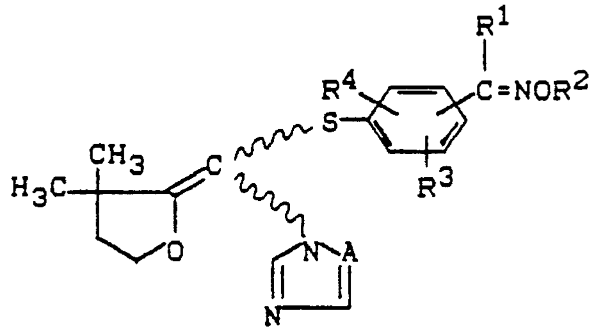

(Ia)

in welcher
A, $R^1$, $R^2$, $R^3$ und $R^4$ die oben angegebene Bedeutung haben,
gegebenenfalls in Gegenwart eines Verdünnungsmittels oxidiert,
oder
c) 2-Chlormethylen-tetrahydrofuran der Formel

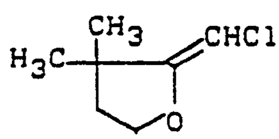

(VIII)

mit (Thio)Phenolen der Formel

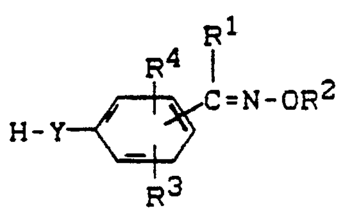

(VII)

in welcher
$R^1$, $R^2$, $R^3$, $R^4$ und Y die oben angegebene Bedeutung haben,
gegebenenfalls in Gegenwart eines Säurebindemittels und gegebenenfalls in Gegenwart eines Verdünnungsmittels umsetzt und danach die so erhaltenen (Thio)Phenoxytetrahydrofuran-2-yliden-methane der Formel

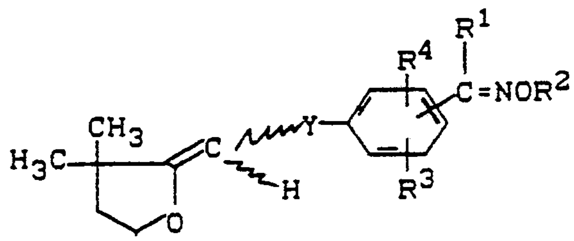

(IX)

in welcher
$R^1$, $R^2$, $R^3$, $R^4$ und Y die oben angegebene Bedeutung haben,
mit Brom in Gegenwart eines Verdünnungmittels umsetzt und die dabei entstehenden (Thio)Phenoxy-tetrahydrofuran-2-yliden-methane der Formel

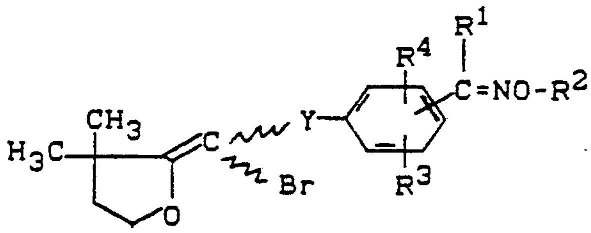

(X)

in welcher
$R^1$, $R^2$, $R^3$, $R^4$ und Y die oben angegebene Bedeutung haben,
mit Azolen der Formel

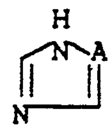

(III)

in welcher
A die oben angegebene Bedeutung hat,
gegebenenfalls in Gegenwart eines Verdünnungsmittels und gegebenenfalls in Gegenwart eines Säurebindemittels umsetzt und gegebenenfalls die so erhaltenen Azolyl-tetrahydrofuran-2-yliden-methane der Formel

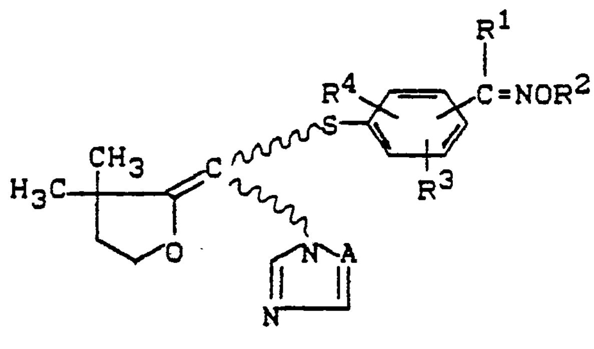

(Ia)

in welcher
A, $R^1$, $R^2$, $R^3$ und $R^4$ die oben angegebene Bedeutung haben,
gegebenenfalls in Gegenwart eines Verdünnungsmittels oxidiert,
und gegebenenfalls anschließend an die so erhaltenen Verbindungen der Formel (I) eine Säure oder ein Metallsalz addiert.

4. Fungizide Mittel, gekennzeichnet, durch einen Gehalt an mindestens einem Azolyl-tetrahydrofuran-2-yliden-methan der Formel (I) gemäß Anspruch 1 bzw. an einem Säureadditions-Salz oder Metallsalz-Komplex eines Azolyl-tetrahydrofuran-2-yliden-methans der Formel (I).

5. Verwendung von Azolyl-tetrahydrofuran-2-yliden-methanen der Formel (I) gemäß Anspruch 1 bzw. von deren Säureadditions-Salzen und Metallsalz-Komplexen zur Bekämpfung von Pilzen.

6. Verfahren zur Bekämpfung von Pilzen, dadurch gekennzeichnet, daß man Azolyl-tetrahydrofuran-2-yliden-methane der Formel (I) gemäß Anspruch 1 bzw. deren Säureadditions-Salze oder Metallsalz-Komplexe auf Pilze und/oder deren Lebensraum ausbringt.

7. Verfahren zur Herstellung von fungiziden Mitteln, dadurch gekennzeichnet, daß an Azolyl-tetrahydrofuran-2-yliden-methane der Formel (I) gemäß Anspruch 1 bzw. deren Säureadditions-Salze oder Metallsalz-Komplexe mit Streckmitteln und/oder oberflächenaktiven Mitteln vermischt.

8. Halogen-(thio)ether-ketone der Formel

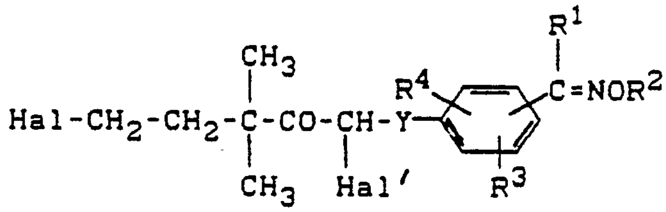

(II)

in welcher
$R^1$ für Wasserstoff oder Alkyl steht,
$R^2$ für Wasserstoff oder Alkyl steht und
$R^3$ und $R^4$ unabhängig voneinander für Wasserstoff, Halogen, Alkyl, Alkoxy, Alkylthio, Halogenalkyl, Halogenalkoxy, Halogenalkylthio, Alkoxycarbonyl, Alkoximinoalkyl, Nitro, Cyano, gegebenenfalls durch Halogen und/oder Alkyl substituiertes Phenyl oder für gegebenenfalls durch Halogen und/oder Alkyl substituiertes Phenoxy stehen,
Y für Sauerstoff oder Schwefel steht und
Hal und Hal′ für Halogen stehen.

9. Verfahren zur Herstellung von Halogen-(thio)etherketonen der Formel

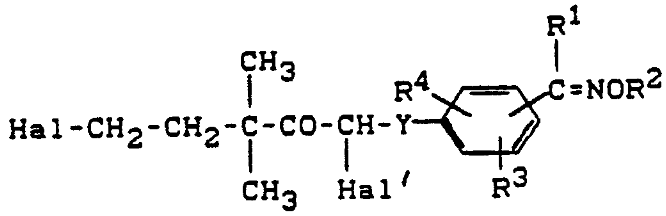

(II)

in welcher
$R^1$ für Wasserstoff oder Alkyl steht,
$R^2$ für Wasserstoff oder Alkyl steht und
$R^3$ und $R^4$ unabhängig voneinander für Wasserstoff, Halogen, Alkyl, Alkoxy, Alkylthio, Halogenalkyl, Halogenalkoxy, Halogenalkylthio, Alkoxycarbonyl, Alkoximinoalkyl, Nitro, Cyano, gegebenenfalls durch Halogen und/oder Alkyl substituiertes Phenyl oder für gegebenenfalls durch Halogen und/oder Alkyl substituiertes Phenoxy stehen,
Y für Sauerstoff oder Schwefel steht und
Hal und Hal′ für Halogen stehen,
dadurch gekennzeichnet, daß man Halogenketone der Formel

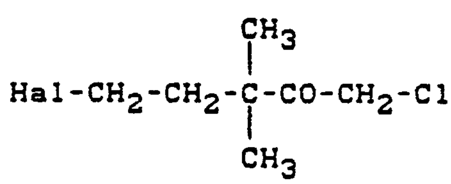

(IV)

in welcher
Hal die oben angegebene Bedeutung hat,
mit (Thio)Phenolen der Formel

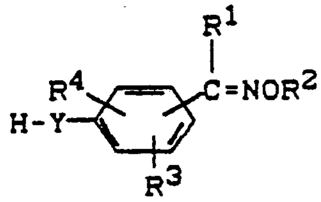

(VII)

in welcher
$R^1$, $R^2$, $R^3$, $R^4$ und Y die oben angegebene Bedeutung haben, gegebenenfalls in Gegenwart eines Säurebindemittels und gegebenenfalls in Gegenwart eines Verdünnungsmittels umsetzt und die dabei entstehenden (Thio)Etherketone der Formel

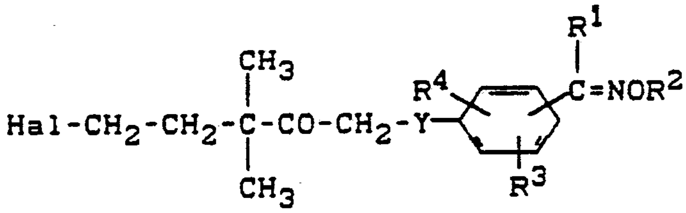

(XI)

in welcher
$R^1$, $R^2$, $R^3$, $R^4$, Hal und Y die oben angegebene Bedeutung haben, mit Halogen in Gegenwart eines Verdünnungsmittels umsetzt.

10. (Thio)Phenoxy-tetrahydrofuran-2-yliden-methane der Formel

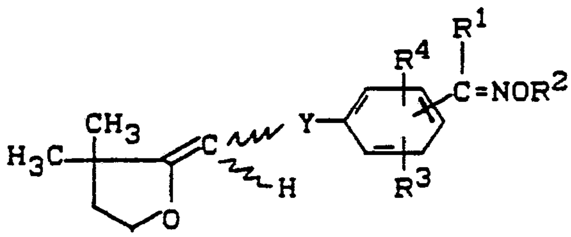

(IX)

in welcher
Y für Sauerstoff oder Schwefel steht,
$R^1$ für Wasserstoff oder Alkyl steht,
$R^2$ für Wasserstoff oder Alkyl steht und
$R^3$ und $R^4$ unabhängig voneinander für Wasserstoff, Halogen, Alkyl, Alkoxy, Alkylthio, Halogenalkyl, Halogenalkoxy, Halogenalkythio, Alkoxycarbonyl, Alkoximinoalkyl, Nitro, Cyano, gegebenenfalls durch Halogen und/oder Alkyl substituiertes Phenyl oder für gegebenenfalls durch Halogen und/oder Alkyl substituiertes Phenoxy stehen.